(19) Europäisches Patentamt
European Patent Office
Office européen des brevets

(11) Publication number: **0 069 481**
**A1**

(12) # EUROPEAN PATENT APPLICATION

(21) Application number: 82303055.6

(22) Date of filing: 14.06.82

(51) Int. Cl.[3]: C 07 D 451/04
C 07 D 451/02, C 07 D 451/14
A 61 K 31/46
//C07D319/18

(30) Priority: 17.06.81 GB 8118603
17.06.81 GB 8118604

(43) Date of publication of application: 12.01.83 Bulletin 83/2

(84) Designated Contracting States: CH DE FR GB IT LI NL

(71) Applicant: BEECHAM GROUP PLC
Beecham House Great West Road
Brentford Middlesex(GB)

(72) Inventor: Watts, Eric Alfred
217 Waterhouse Moor
Harlow Essex(GB)

(74) Representative: Stott, Michael John et al,
European Patent Attorney Beecham Pharmaceuticals
Great Burgh Yew Tree Bottom Road
Epsom Surrey KT18 5XQ(GB)

(54) **Azabicycloalkyl benzamides, process for their preparation and pharmaceutical compositions containing them.**

(57) Compounds of the formula (I), and pharmaceutically acceptable salts and N-oxides thereof, and solvent adducts of any of the foregoing:

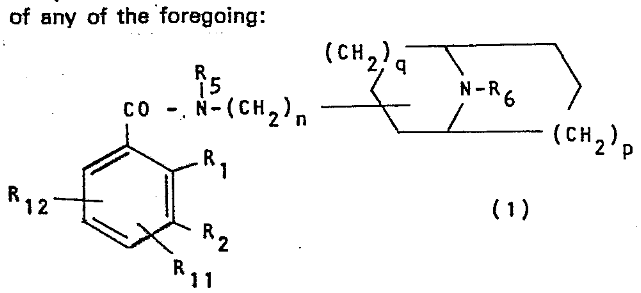

(1)

wherein

n, p and q are independently 0 to 2.

$R_5$ is hydrogen of $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and

either

$R_1$ is $C_{1-6}$ alkoxy or $C_{1-6}$ alkylthio and one of $R_2$, $R_{11}$ and $R_{12}$ is $C_{1-6}$ alkylsulphinyl and the other two together are $C_{1-2}$ alkylenedioxy, or one of the other two is hydrogen and the other is selected from halogen trifluoromethyl, $C_{1-6}$ alkyl, $C_{1-6}$ alkoxy, $C_{1-7}$ acyl, hydroxy or amino substituted by or aminocarbonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phen $C_{1-4}$ alkyl groups or by $C_{4-5}$ polymethylene;

or

$R_1$ and $R_2$ together are $C_{1-2}$ alkylenedioxy, $R_{12}$1 is $C_{1-6}$ alkylsulphinyl and $R_{11}$ is selected from the class of hydrogen, halogen trifluoromethyl, $C_{1-6}$ alkyl, $C_{1-6}$ alkoxy, $C_{1-7}$ acyl, $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phen $C_{1-4}$ alkyl groups or optionally N-disubstituted by $C_{4-5}$ polymethylene, having useful pharmacological activity, a process for their preparation, and pharmaceutical compositions containing them.

EP 0 069 481 A1

Croydon Printing Company Ltd.

TITLE MODIFIED
see front page

ACTIVE COMPOUNDS

This invention relates to novel compounds, to pharmaceutical compositions containing them, and to a process for their preparation.

European Patent Application No 79302978.6 and allowed U.S. Patent Application No. 107,413 disclose that compounds of the formula (A), and pharmaceutically acceptable salts thereof:

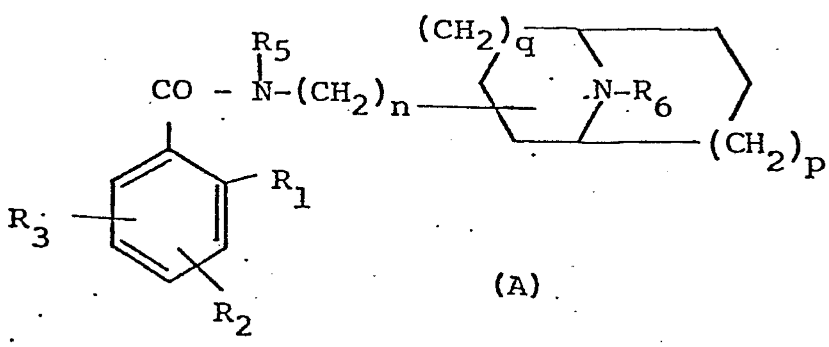

(A)

wherein:

$R_1$ is a $C_{1-6}$ alkoxy group;

$R_2$ and $R_3$ are the same or different and are hydrogen, halogen, $CF_3$, $C_{2-7}$ acyl, $C_{2-7}$ acylamino, or amino, aminocarbonyl or aminosulphone optionally substituted by one or two $C_{1-6}$ alkyl groups, $C_{1-6}$ alkylsulphone or nitro ;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen; and

n, p and q are independently 0 to 2; have useful pharmacological activity. More specifically the compounds of formula (A) are stated to be useful in the treatment of disorders related to impaired gastro-intestinal motility and/or in the treatment of disorders of the central nervous system. All the compounds are stated to have anti-emetic activity.

The said European and US Patent Applications, the subject matter of which is imported herein by reference, has extensive exemplification of typical compounds of the formula (A) and of their pharmacological activity, establishing the veracity of the claimed utilities for the class of compounds defined by formula (A).

In the said formula (A) $R_3$ may be a $C_{1-6}$ alkylsulphonyl group ($R-SO_2-$). It has now been discovered that certain compounds of a generally similar structure to that of formula (A) but wherein $R_3$ is a $C_{1-6}$ alkylsulphinyl group ($R-SO-$), also have useful pharmacological activity.

Accordingly the present invention provides a compound of the formula (I), and pharmaceutically acceptable salts and N-oxides thereof, and solvent adduct of any of the foregoing:

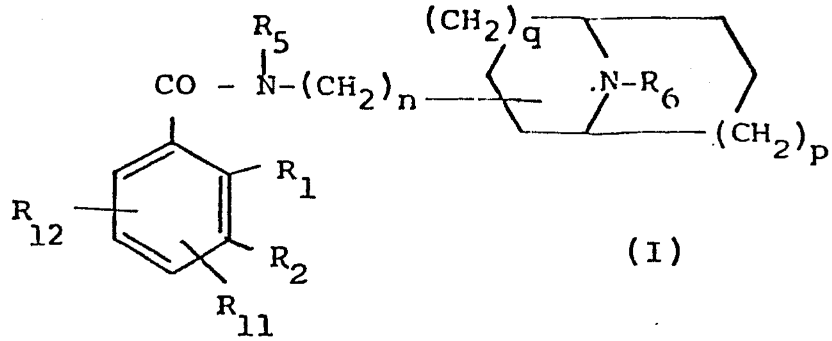

(I)

n, p and q are independently 0 to 2.

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and

either

$R_1$ is $C_{1-6}$ alkoxy or $C_{1-6}$ alkylthio and one of $R_2$,$R_{11}$ and $R_{12}$ is $C_{1-6}$ alkylsulphinyl and the other two together are $C_{1-2}$ alkylenedioxy, or one of the other two is hydrogen and the other is selected from halogen,trifluoromethyl,$C_{1-6}$ alkyl,$C_{1-6}$alkoxy,$C_{1-7}$acyl,hydroxy or amino substituted by or aminocarbonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phen $C_{1-4}$ alkyl groups or optionally N-disubstituted by $C_{4-5}$ polymethylene;

or

$R_1$ and $R_2$ together are $C_{1-2}$ alkylenedioxy, $R_{12}$ is $C_{1-6}$ alkylsulphinyl and $R_{11}$ is selected from the class of hydrogen, halogen trifluoromethyl, $C_{1-6}$ alkyl, $C_{1-6}$ alkoxy, $C_{1-7}$ acyl, $C_{1-7}$ acylamino, $C_{1-6}$ alkylsulphonyl, $C_{1-6}$ alkylsulphinyl, hydroxy, nitro or amino,or aminocarbonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phen $C_{1-4}$ alkyl or optionally N-disubstituted by $C_{4-5}$ polymethylene.

A group of compounds within formula (I) is of formula (IA):

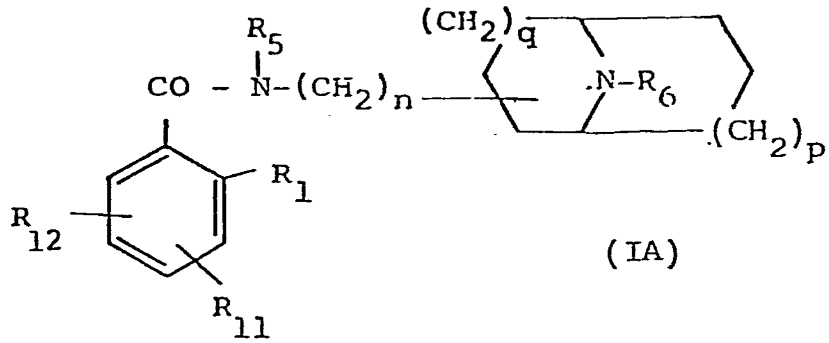

(IA)

wherein:

$R_1$ is $C_{1-6}$ alkoxy or $C_{1-6}$ alkylthio;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and

$R_{11}$ is halogen, $CF_3$, $C_{1-7}$ acyl, aminocarbonyl optionally substituted by one or two $C_{1-6}$ alkyl groups, $C_{1-6}$ alkoxy, $C_{1-6}$ alkylthio or hydroxy;

$R_{12}$ is $C_{1-6}$ alkylsulphinyl; and

n, p and q are independently 0 to 2.

A second group of compounds within formula (I) is of formula (IB):

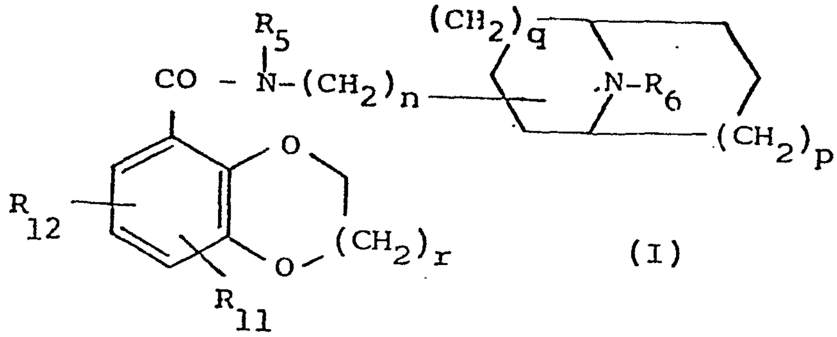

(I)

wherein:

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group;

$R_{11}$ is hydrogen, halogen, $CF_3$, $C_{1-7}$ acyl, $C_{1-7}$ acylamino or amino, aminocarbonyl optionally substituted by one or two $C_{1-6}$ alkyl groups, $C_{1-6}$ alkoxy, $C_{1-6}$ alkylthio, hydroxy or nitro;

$R_{12}$ is $C_{1-6}$ alkylsulphinyl; and
n, p, q and r are independently 0 to 2.

r is favourably 0 or 1, preferably 1.

In formula (I), examples of $C_{1-6}$ alkylsulphinyl groups include methyl, ethyl and n- and iso-propylsulphinyl, favourably methyl- or ethyl-, in particular methyl-sulphinyl.

It is generally preferred that such a group is in 5-position relative to azabicyclylaminocarbonyl side-chain taken as 1.

Favourably it is 5-methyl or 5-ethyl-sulphinyl, preferably 5-methylsulphinyl.

Preferred examples of $R_1$ include methoxy, ethoxy, n- and iso- propoxy, methylthio, ethylthio, n- and iso-propylthio. Most preferably, $R_1$ is methoxy.

In such compounds, one of $R_2$ and $R_{11}$ is $C_{1-6}$ alkylsulphinyl, the other two may suitably together be methylenedioxy or ethylenedioxy or one is hydrogen and the other is selected from chlorine, bromine and amino substituted by one or two methyl, ethyl, n- or iso-propyl, n-, sec- or tert-butyl, cyclohexyl, phenyl or benzyl groups or disubstituted by $C_4$ or $C_5$ polymethylene, methoxy, ethoxy, n- and iso-propoxy, methyl, ethyl and n- and iso-propyl.

Preferably, the other one is chloro, bromo or methoxy.

Alternatively, $R_1$ and $R_2$ together are methylenedioxy or ethylenedioxy and $R_{11}$ suitably may be hydrogen, chlorine, bromine, amino, $C_{1-4}$ alkanoylamino such as formylamino, acetylamino, propionylamino, n- and iso-butyrylamino, and amino substituted by one or two methyl, ethyl, n- or iso-propyl, n-, sec- or tert-butyl groups, cyclohexyl, phenyl or benzyl groups or N-disubstituted by $C_4$ or $C_5$ polymethylene, nitro, methoxy, ethoxy, n- and iso-propoxy, methyl, ethyl, or n- or iso-propyl.

Preferably $R_{11}$ is hydrogen, chloro or amino. Often, $R_{11}$ is hydrogen.

Suitable examples of $R_6$ when $C_{1-4}$ alkyl include methyl, ethyl, n- and iso-propyl and n-, sec-, iso- and tert-butyl, particularly methyl, n-propyl and sec-butyl.

Similarly, within $C_{1-7}$ radicals, $C_{5-7}$ alkyl are also of interest.

Suitable examples of $R_6$ when $C_{5-7}$ alkyl include n-pentyl, n-hexyl and n-heptyl, 3-methylbutyl, 4-methylpentyl and 5-methylhexyl.

When $R_6$ is a group $-(CH_2)_sR_7$ as defined, suitable examples of $R_7$ include $C_{5-8}$ cycloalkyl, preferably cyclohexyl. s is preferably 1.

When $R_6$ is a group $-(CH_2)_t\ R_8$ as defined, t is preferably 1.

In such a group $R_6$, when $R_8$ is $C_{2-5}$ alkenyl, suitable examples thereof include vinyl, prop-1-enyl, prop-2-enyl, 1-methylvinyl, but -1-enyl, but-2-enyl, but-3-enyl, 1-methylenepropyl, 1-methylprop-1-enyl and 1-methylprop-2-enyl, in their E and Z forms where stererisomerism exists.

A preferred $C_{1-5}$ alkenyl $R_8$ radical is vinyl, so that $R_6$ is preferably allyl.

When $R_8$ is optionally substituted phenyl as defined above, suitable examples of such optional phenyl substituents include methyl, ethyl, n- and iso-propyl, n, sec- and tert-butyl; methoxy, ethoxy, n- and iso-propoxy; $CF_3$, fluoro, chloro or bromo. Preferably $R_8$ when optionally substituted phenyl is unsubstituted. When $R_8$ is thienyl it may be 2- or 3-thienyl, generally 2-thienyl.

Two values for $R_6$ are optionally substituted benzyl as hereinbefore defined and thienylmethyl (also called thenyl).

Where $R_6$ is $C_{1-4}$alkyl, the preferred examples of $C_{1-4}$ alkyl include metal, ethyl, n- and iso propyl, and n-, iso-, sec- and tert- butyl; methyl however is most preferred.

The most preferred examples of $R_6$, when $-(CH_2)_tR_{11}$, are those wherein t is 1 and $R_{11}$ is unsubstituted phenyl or monosubstituted phenyl in particular mono-p-substituted phenyl. Examples of preferred p- substituents include methyl, trifluoromethyl, fluoro, chloro and bromo, especially fluoro. Unsubstituted benzyl, p-fluorobenzyl, p-chlorobenzyl and p-methylbenzyl are especially preferred examples of $R_6$.

n is preferably 0. q is suitably 0 to 1, preferably 1. p is suitably 0 to 1, preferably 0.

Often the amide and side chain nitrogen atoms are separated by a minimum of 2 or 3 carbon atoms, preferably 3.

When separation is 3 atoms and n is 0, the $CONR_5$ moiety is preferably in an equatorial orientation to the bicyclic system.

The pharmaceutically acceptable salts of the compounds of the formula (I) include acid addition salts with conventional acids such as hydrochloric, hydrobromic, phosphoric, sulphuric, citric, tartaric, lactic and acetic acid and the like.

The pharmaceutically acceptable salts of the compounds of the formula (I) also include quaternary ammonium salts. Examples of such salts include such compounds quaternised by compounds such as $R_9$ - Y wherein $R_9$ is $C_{1-6}$ alkyl, phenyl - $C_{1-6}$ alkyl or $C_{5-7}$ cycloalkyl, and Y is an anion of an acid. Suitable examples of $R_9$ include methyl, ethyl and n- and iso-propyl; and benzyl and phenylethyl. Suitable examples of Y include the halides such as chloride, bromide and iodide.

The compounds of the formula (I) and their salts and N-oxides may also form solvent adducts, and the invention extends to such solvent adducts.

From the aforesaid it will be seen that in one aspect the moiety of the formula (II):

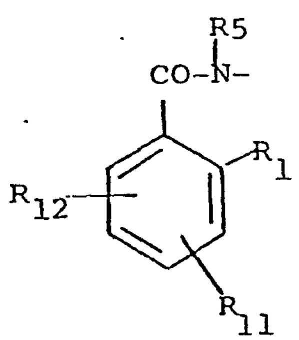

(II)

in a compound of the formula (I) will have the structure (III):

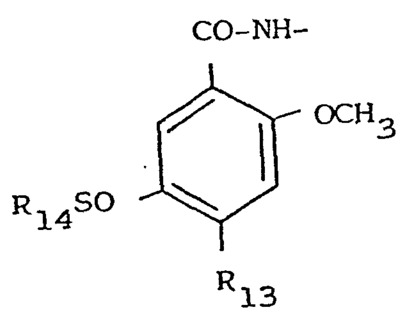

(III)

wherein $R_{14}$ is $C_{1-6}$ alkyl and $R_{13}$ is halogen.
Suitable $R_{14}SO$ are as described for $R_{12}$ under formula (1).

Preferably $R_{13}$ is chloro. Preferably $R_{14}$ is methyl or ethyl, in particular methyl.

In a group of compounds within those of formula (I) the moiety of formula (II) will be of the formula (III), and the moiety of formula (IV):

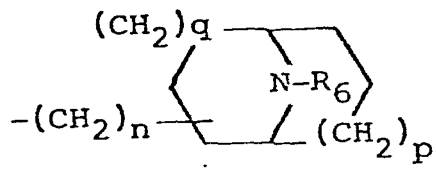

(IV)

will have the formula (V):

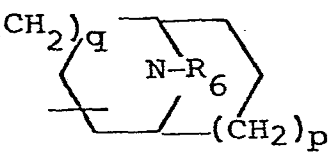

(V)

wherein the variables are as defined in formula (I), so that these compounds of the formula (I) are of the formula (VI):

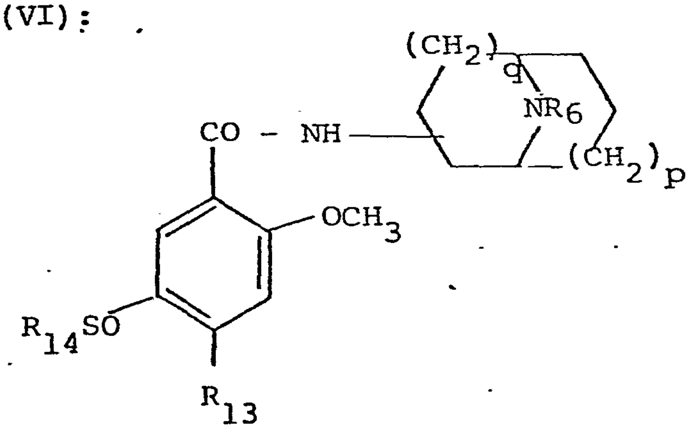

(VI)

wherein the variables are as defined above.

Suitable and preferred $R_{14}$ are as so described under formula (III).

More suitably p is 0 or 1, it is believed preferably 0. Preferably q is 1 and the moiety of formula (III) is then attached at the 3-position (conventional numbering) and in the β orientation.

Suitable and preferred examples of $R_6$ in formula (VI) include those listed under formula (I) for $R_6$, examples of $R_6$ include $C_{1-7}$ alkyl and cyclohexylmethyl. Particularly preferred examples of $R_6$ include benzyl optionally substituted in the phenyl ring as defined under formula (I).

A sub-group of compounds within those of formula (VI) are those of the formula (VII):

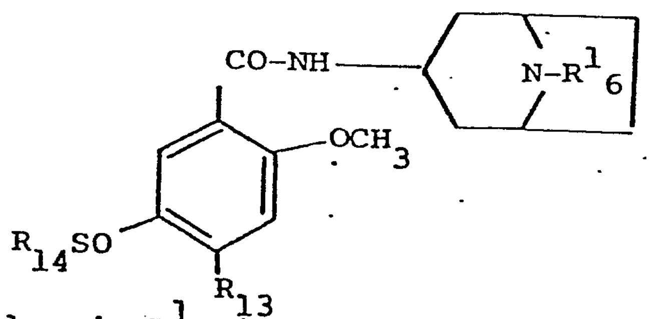

(VII)

wherein $R^1_6$ is $C_{1-4}$ alkyl.

Suitable examples of $R^1_6$ are as so described for $R_6$ $C_{1-4}$ alkyl under formula (I).

It is preferred that the moiety of the formula (III) is in the β-orientation to the nortropane ring.

A preferred sub-group of compounds within those of formula (VI) are those of the formula (VIII):

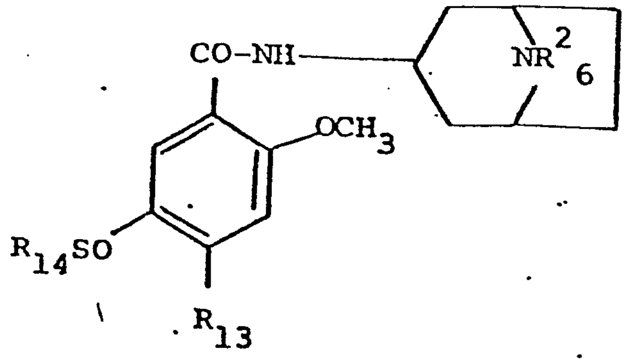

(VIII)

wherein $R^2_6$ is $C_{5-7}$ alkyl; a group $-(CH_2)_tR^1_8$ wherein t is 1 or 2 and $R^1_8$ is optionally substituted phenyl as defined in formula (I); cyclohexylmethyl, or thienylmethyl.

Suitable and preferred $R^2_6$ are as so described for the corresponding $R_6$ groups under formula (I).

$R^2_6$ benzyl is especially preferred.

It is preferred that the moiety of the formula (III) is in the β-orientation to the nortropane ring.

A sub-group of compounds within those of the formula (VI) of interest are those of the formula (IX):

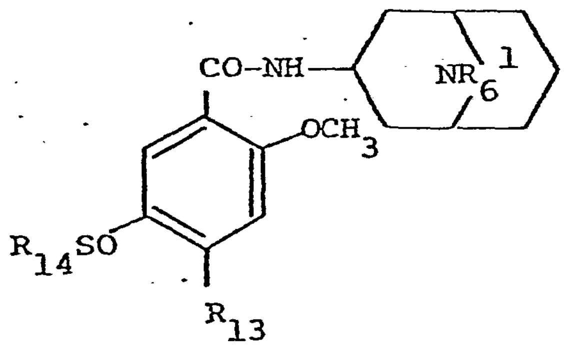

(IX)

wherein $R_6^1$ is as defined in formula (VII).

Suitable examples of $R^1_6$ are as so described under formula (VII).

Another sub-group of compounds within those of the formula (VI) of interest are those of the formula (X):

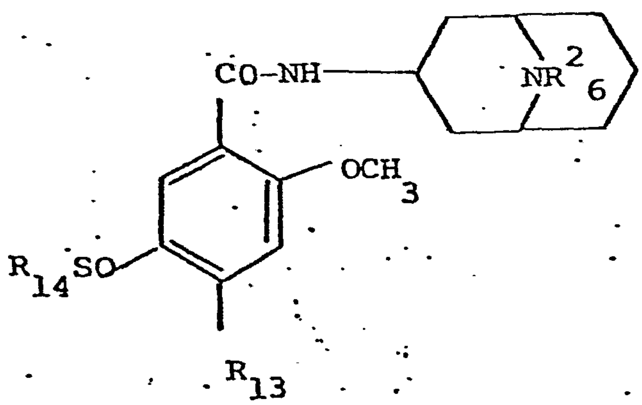

(X)

wherein $R^2_6$ is as defined in formula (VIII).

Suitable and preferred examples of $R^2_6$ are as so described under formula (VIII).

A second preferred group of compounds within those of the formula (I) which is of interest is of the formula (XI):

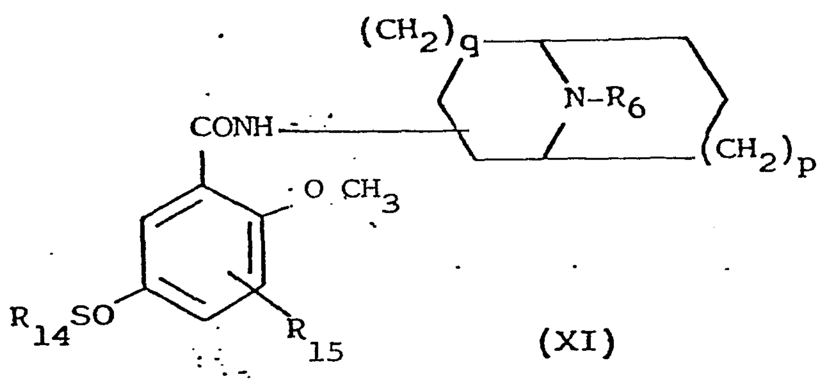

(XI)

wherein $R_{14}$ and $R_6$ are as defined in formula (VI), and $R_{15}$ is $C_{1-6}$ alkoxy or hydroxy.

It is preferred that the CONH moiety is in the β-orientation to the nortropane ring.

Suitable $R_{15}$ are as so described for alkoxy $R_2/R_{11}$ groups under formula (I).

$R_{15}$ is preferably methoxy.

The 2,3-dimethoxy and 2,4-dimethoxy nuclei (with respect to -CONH- taken as 1) are particularly preferred.

Suitable and preferred $R^1_6$ are as so described under formula (VI).

A sub-group of compounds within those of formula (XI) of interest is of formula (XII):

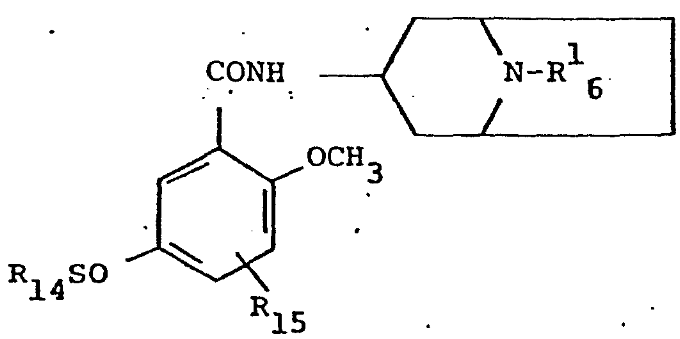

(XII)

wherein $R^1_6$ is as defined in formula (VII) and $R_{14}$ and $R_{15}$ are as defined in formula (XI).

Suitable and preferred $R^1_6$ are as so described under formula (VII). Suitable and preferred $R_{14}$ and $R_{15}$ are so described under formula (XI).

A second sub-group of compounds within those of formula (XI) of interest is of formula (XIII):

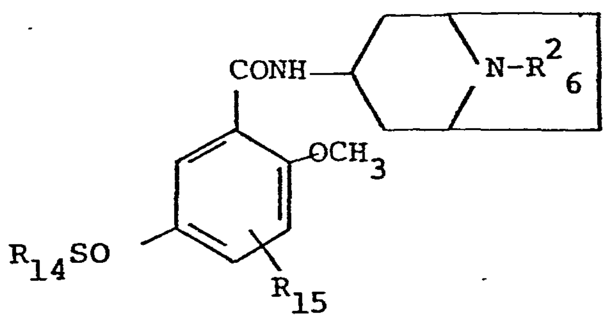

(XIII)

wherein $R^2_6$ is as defined in formula (VIII) and $R_{14}$ and $R_{15}$ are as defined in formula (XI).

Suitable and preferred $R^2_6$ are as so described under formula (VIII). Suitable and preferred $R_{14}$ and $R_{15}$ are as so defined under formula (XI).

A third sub-group of compounds within those of formula (XI) of interest is of formula (XIV):

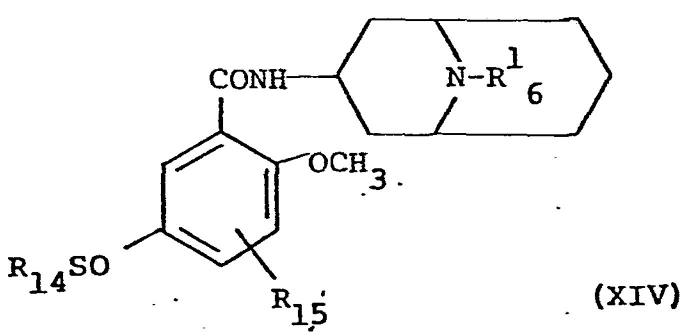

(XIV)

wherein $R^1_6$ is as defined in formula (VII) and $R_{14}$ and $R_{15}$ are as defined in formula (XI).

Suitable and preferred $R^1_6$ are as so described under formula (VII). Suitable and preferred $R_{14}$ and $R_{15}$ are as so described under formula (XI).

A fourth sub-group of compounds within those of formula (XI) of interest is of formula (XV):

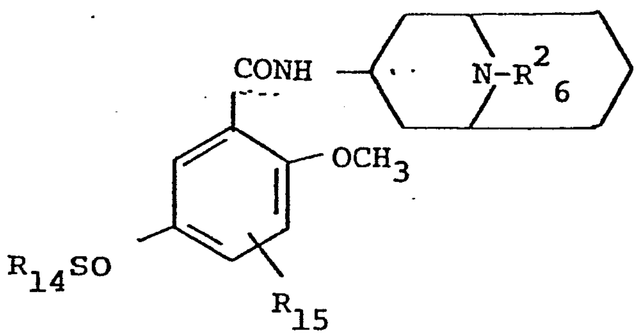

(XV)

wherein $R^2_6$ is as defined in formula (VIII) and $R_{14}$ and $R_{15}$ are as defined in formula (XI).

Suitable and preferred $R^2_6$ are as so described under formula (VIII). Suitable and preferred $R_{14}$ and $R_{15}$ are as defined in formula (XI).

A third group of compounds within those of formula (I) is of formula (XVI):

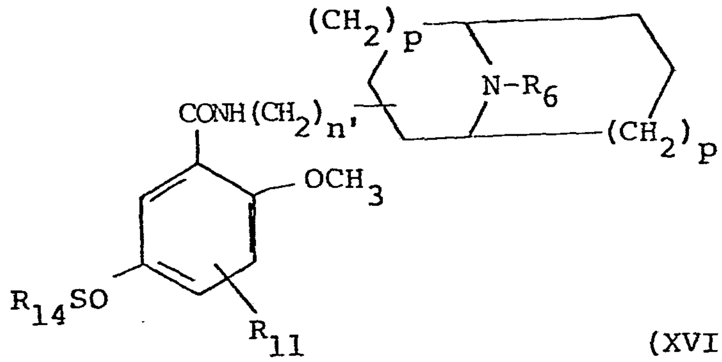

(XVI)

wherein n' is 1 or 2 and the remaining variables are as defined in formula (VI).

More suitably p and q are each 0 or 1.

Preferably p is 0 and q is 1. Preferably n is 1. Suitable and preferred $R_6$, $R_{11}$ and $R_{14}$ are as so described under formula (VI).

In another aspect the moiety of formula (II):

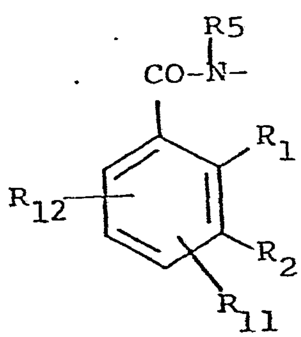

(II)

in a compound of the formula (I) will have the structure (III):

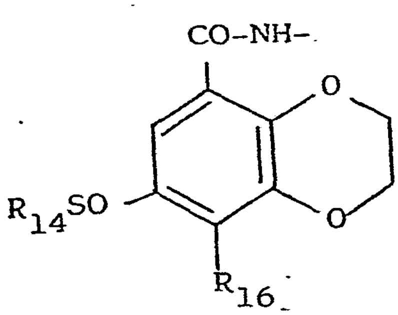

(XVII)

wherein $R_{16}$ is amino, chloro or alkoxy.

$R_{14}$ is $C_{1-6}$ alkyl.

Preferably $R_{14}$ is methyl or ethyl, in particular methyl.

A fourth group of compounds within those of formula (I) and pharmaceutically acceptable salts thereof, are thus of the formula (XVIII).

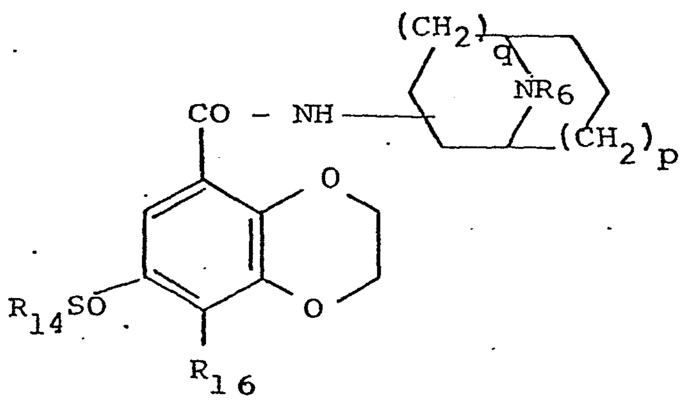

(XVIII)

wherein the variables are as defined above.

Suitable and preferred $R_{14}$ are as so described under formula (III).

More suitably p is 0 or 1, it is believed preferably 0. Preferably q is 1 and the moiety of formula (III) is then attached at the 3-position (conventional numbering) and in the β orientation.

Suitable and preferred examples of $R_6$ in formula (XVIII) include those listed under formula (I) for $R_6$. Particularly preferred examples of $R_6$ include $C_{1-7}$ alkyl and cyclohexylmethyl. Particularly preferred examples of $R_6$ also include benzyl optionally substituted in the phenyl ring as defined under formula (I).

A sub-group of compounds within those of formula (XVIII) are those of the formula (XIX):

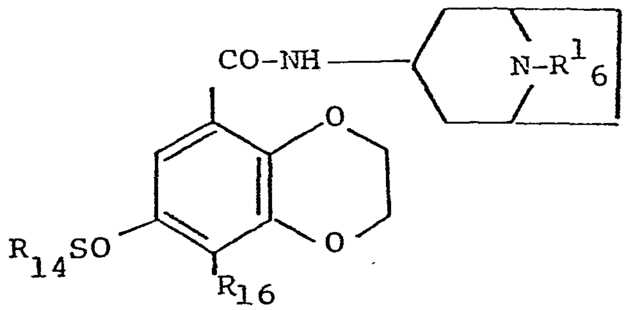

(XIX)

wherein $R^1_6$ is $C_{1-4}$ alkyl.

Suitable examples of $R^1_6$ are as so described under formula (VII)

It is preferred that the moiety of the formula (XVII) is in the β-orientation to the nortropane ring.

A preferred sub-group of compounds within those of formula (XVIII) are those of the formula (XX):

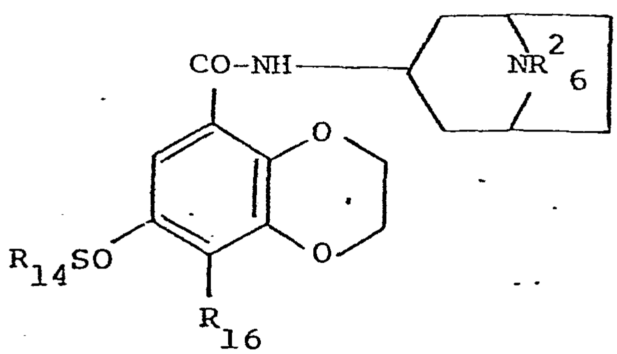

(XX)

wherein $R^2_6$ is $C_{5-7}$ alkyl; a group $-(CH_2)_tR^1_8$ wherein t is 1 or 2 and $R^1_8$ is optionally substituted phenyl as defined in formula (I); cyclohexylmethyl, or thienylmethyl.

Suitable and preferred $R^2_6$ are as so described for the corresponding $R_6$ groups under formula (I).

$R^2_6$ benzyl is especially preferred.

It is preferred that the moiety of the formula (XVII) is in the β-orientation to the nortropane ring.

A sub-group of compounds within those of the formula (VI) of interest are those of the formula (IX):

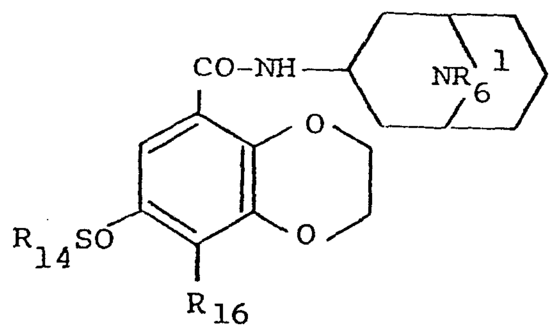

(XXI)

wherein $R_6^1$ is as defined in formula (VII).

Suitable examples of $R_6^1$ are as so described under formula (VII).

Another sub-group of compounds within those of the formula (VI) of interest are those of the formula (X):

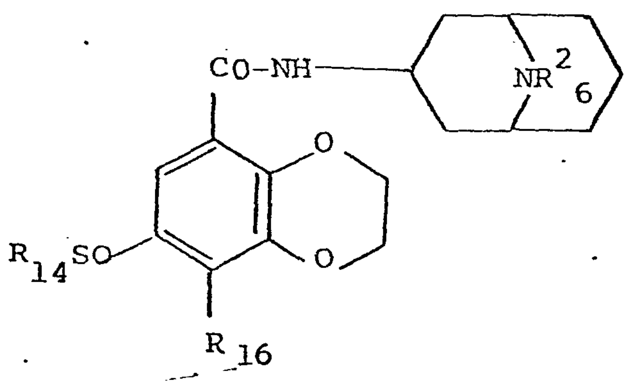

(XXII)

wherein $R^2_6$ is as defined in formula (VIII).

Suitable and preferred examples of $R^2_6$ are as so described under formula (VIII).

A fifth preferred group of compounds within those of the formula (I) which is of interest is of the formula (XXIII):

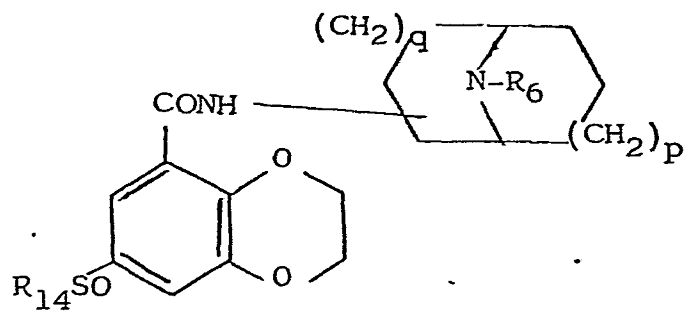

(XXIII)

wherein $R_{14}$ and $R_6$ are as defined in formula (VI).

It is preferred that the CONH moiety is in the β-orientation to the nortropane ring.

Suitable and preferred $R_6$ and $R_{14}$ are as so described under formula (VI).

A sub-group of compounds within those of formula (XXIII) of interest is of formula (XXIV).

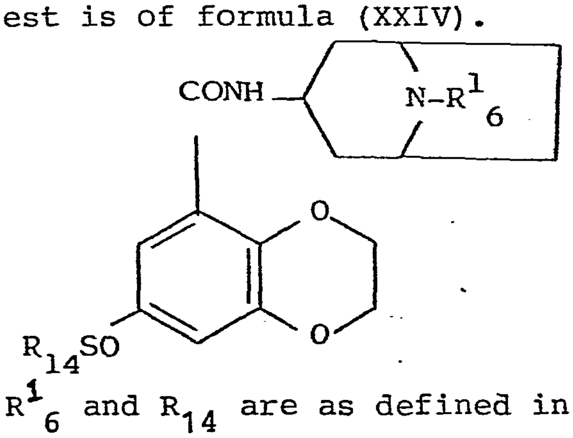

(XXIV)

wherein $R^1_6$ and $R_{14}$ are as defined in formula (VII).

Suitable and preferred $R^1_6$ are as so described under formula (VII). Suitable and preferred $R_{14}$ are so described under formula (XXIII).

A second sub-group of compounds within those of formula (XXIII) of interest is of formula (XIII):

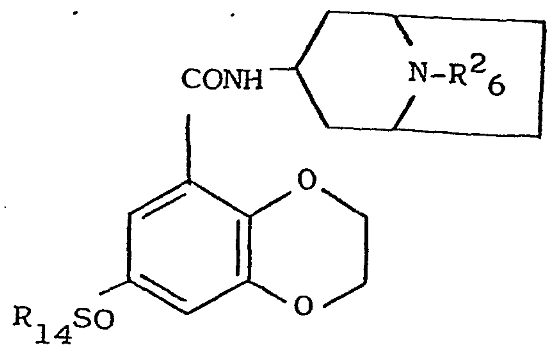

(XXV)

wherein $R^2_6$ and $R_{14}$ are as defined in formula (VIII).

Suitable and preferred $R^2_6$ are as so described under formula (VIII). Suitable and preferred $R_{14}$ are as so defined under formula (XXIII).

A third sub-group of compounds within those of formula (XXIII) of interest is of formula (XXVI).

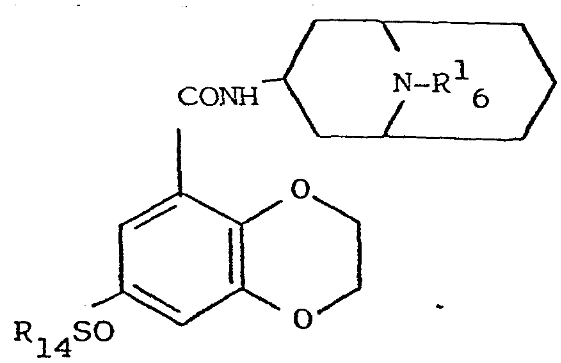

(XXVI)

wherein $R'_6$ and $R_{14}$ are as defined in formula (VII).

Suitable and preferred $R^1_6$ are as so described under formula (VII). Suitable and preferred $R_{14}$ and $R_{15}$ are as so described under formula (XXIII).

A fourth sub-group of compounds within those of formula (XXIII) of interest is of formula (XXVII).

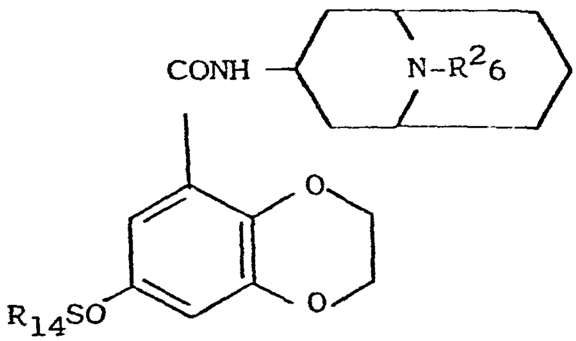

(XXVII)

wherein $R^2_6$ is as defined in formula (VIII) and $R_{14}$ is as defined in formula (III).

Suitable and preferred $R^2_6$ are as so described under formula (VIII). Suitable and preferred $R_{14}$ are as so described under formula (XXIII).

A sixth group of compounds within those of formula (I) is of formula (XXVIII):

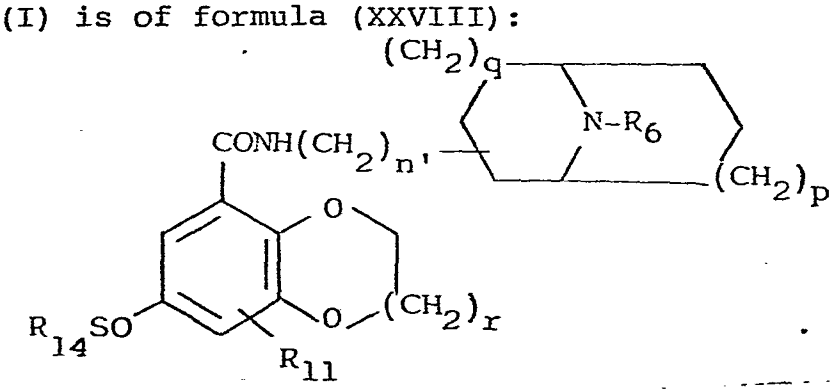

(XXVIII)

wherein n' is 1 or 2, r is 1 or 2 and $R_{14}$ is as defined in formula (III) and $R_{11}$ as in formula (I).

More suitably p and q are each 0 or 1.

Preferably p is 0 and q is 1. Preferably n is 1.

Suitable and preferred $R_6$, $R_{11}$ and $R_{14}$ are as so described under formulae (I) and (III).

It will of course be realised that the compounds of the formula (I) have chiral or prochiral centres, and thus are capable of existing in a number of stereoisomeric forms.

The invention extends to each of these stereoisomeric forms, and to mixtures thereof (including racemates). The different stereoisomeric forms may be separated one from the other by the usual methods, or any given isomer may be obtained by stereospecific or asymmetric synthesis.

The compounds of formula (I) may be prepared in analogous manner to the compounds of formula (A).

The invention thus provides a process for the preparation of a compound of the formula (I), which process comprises reacting an acid of the formula (XXIX):

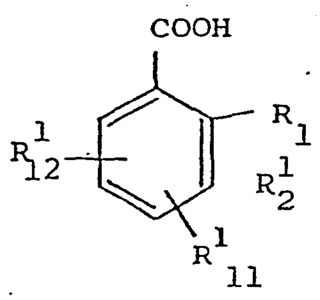

(XXIX)

wherein $R_1$ is as defined; and $R^1_2$, $R^1_{11}$ and $R^1_{12}$ are $R_2$, $R_{11}$ and $R_{12}$ as defined but when $R_1$ is $C_{1-6}$ alkoxy the third of them may be $C_{1-6}$ alkylthio; or a reactive derivative thereof, with a compound of formula (XXX).

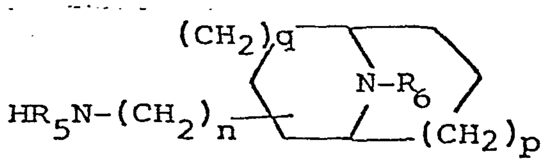

(XXX)

wherein $R_6$ is as defined in formula (I), the remaining variable groups being as defined in formula (I); and thereafter if necessary converting a group $R^1_2$, $R^1_{11}$ or $R^1_{12}$ in the thus formed compound to another group $R_2$, $R_{11}$ or $R_{12}$ respectively; converting $R_6$ to another $R_6$; and optionally forming a pharmaceutically acceptable salt of the resultant compound of the formula (I).

'Reactive derivative' when used herein means a derivative of the compound (XIX) which can be reacted with the compound (XXX) to form an amido linkage between the acid group of the compound (XIX) and the amino group of the compound of the formula (XXX).

Often this reactive derivative will be the acid halide, such as the acid chloride, of the acid (XXIX). In such cases, the reaction will normally be carried out in an inert solvent, preferably in the presence of an acid acceptor. The inert solvent can be any solvent inert to both reactants, such as benzene, toluene, diethyl ether or dichloromethane. The acid acceptor is suitably an organic base such as a tertiary amine, e.g. triethylamine, trimethylamine, pyridine or picoline, or an inorganic acid acceptor, such as calcium carbonate, sodium carbonate, potassium carbonate or the like. It should also be noted that it is possible to use certain acid acceptors as the inert solvent, for example organic bases.

These reactions may be carried out at any non-extreme temperature such as $-10^{\circ}$-$100^{\circ}C$ and more suitably $0^{\circ}$-$80^{\circ}C$. The higher reaction temperatures are employed with less active acids of the formula (XXIX) whereas the lower temperatures are employed with the more reactive acids of the formula (XXX).

Another useful reactive derivative of the acid XXIX is a highly activated ester wuch as the pentachlorophenyl ester, when ambient temperatures may be used. The reaction is generally effected in an inert polar solvent, such as dimethylformamide.

The reaction may also be carried out by forming an anhydride of the acid (XXIX) in the usual manner, and reacting that with the compound (XXX); normally a conventional mixed anhydride will be used; or by reacting the acid (XXIX) and the compound (XXX) in the presence of a dehydrating catalyst such as a carbodiimide, for example dicyclohexylcarbodiimide.

The intermediates of the formulae (XXIX) and (XXX) are either known compounds or can be prepared by analogous processes to known compounds.

It will be realised that in the compound of the formula (I) the $-CO-NR_5-(CH_2)_n-$ linkage may have an α or β orientation with respect to the ring of the bicyclic moiety to which it is attached. A mixture of α and β isomers of the compound of the formula (I) may be synthesised non-stereospecifically and the desired isomer separated conventionally therefrom, e.g. by chromatography; or alternatively the α or β isomer may if desired be synthesised from the corresponding α or β form of the compound of the formula (XXX).

Synthesis from the corresponding α or β isomer of the compound of the formula (XXX) is in general preferred.

The α or β form of the compound of the formula (XVIII) may if desired be prepared by known stereospecific processes, such as those leading to the α and β isomers of the compound of the formula (XXX) depicted in the Scheme and described in Descriptions 3C, 4A and 4C of European Patent Application No. 79302978.6 and allowed U.S. Patent Application No. 107,413 for the α-isomers and Descriptions 2 and 3A and B of these Applications for the β-isomers.

The precursor of the compound of the formula (XXX) may be stereospecifically synthesised, such as the azide (D3) of Description 2 of the above European and U.S. Applications and then converted to the corresponding desired isomer of the compound of the formula (XXX) under non-stereospecific conditions with retention of configuration.

Alternatively, the precursor may itself have no (pro)chiral centre at the relevant position, such as the oximes and imines of Descriptions 3 and 4 of the above European and U.S. Applications but be converted under stereospecific conditions to the desired isomer of the compound of the formula (XXX).

Alternatively, a mixture of the α and β isomers of the compound of the formula (XXX) may be synthesised non-stereospecifically and the desired isomer separated conventionally therefrom e.g. by chromatography. However, in this case it is generally more convenient to react the mixture to give a mixture of α and β isomers of the compound of the formula (I) and to separate these if desired as hereinbefore described.

The following Scheme 1 illustrates stereospecific and non-stereospecific synthetic routes to intermediates of the formula (XXX) wherein n is 0.

Scheme 1

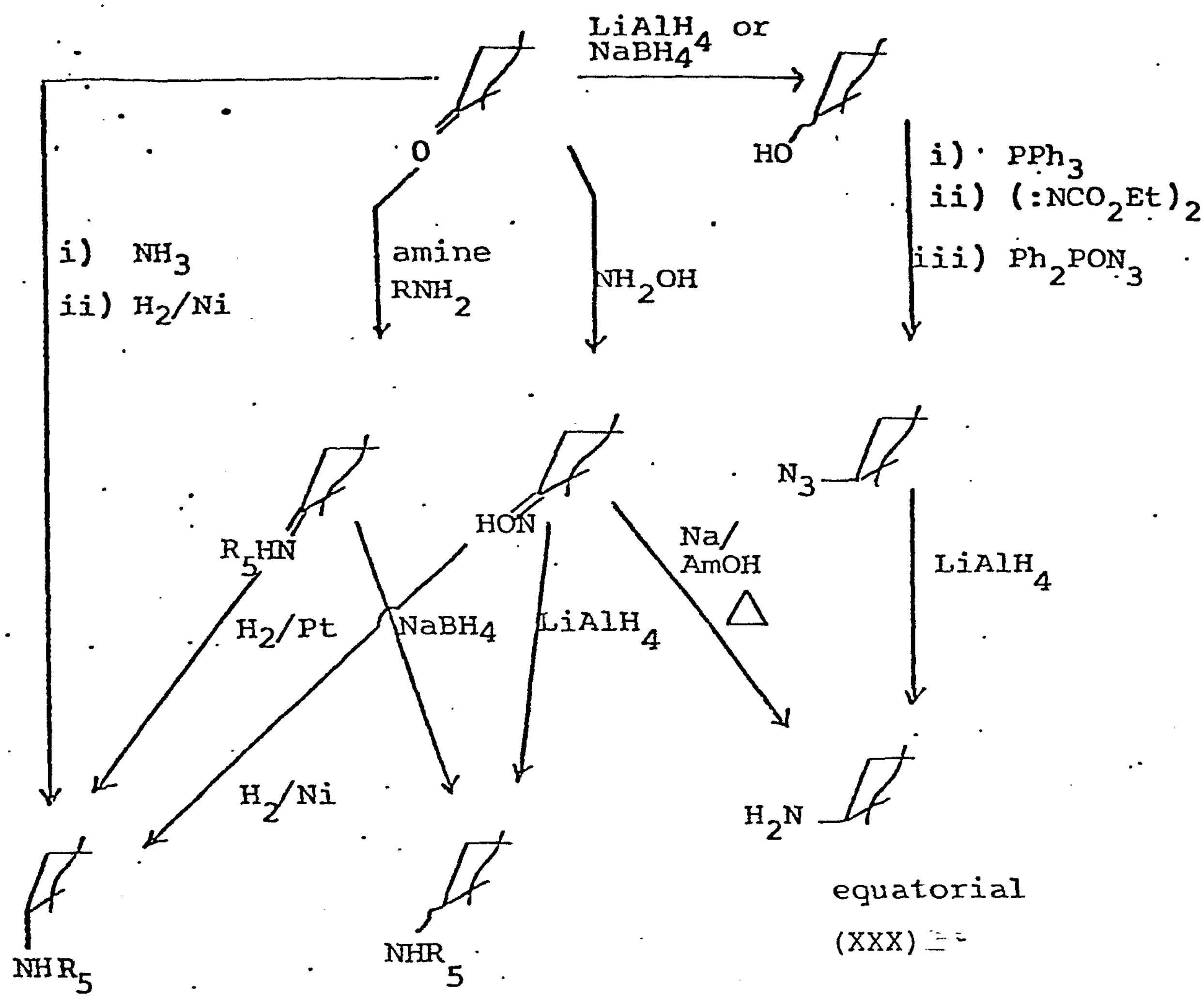


axial (XXX)

axial, equatorial (XXX)

equatorial (XXX)

(Remainder of ring system omitted for clarity)

The following Scheme 2 illustrates preparative routes to intermediates of the formula (XXX) wherein n is 1 or 2.

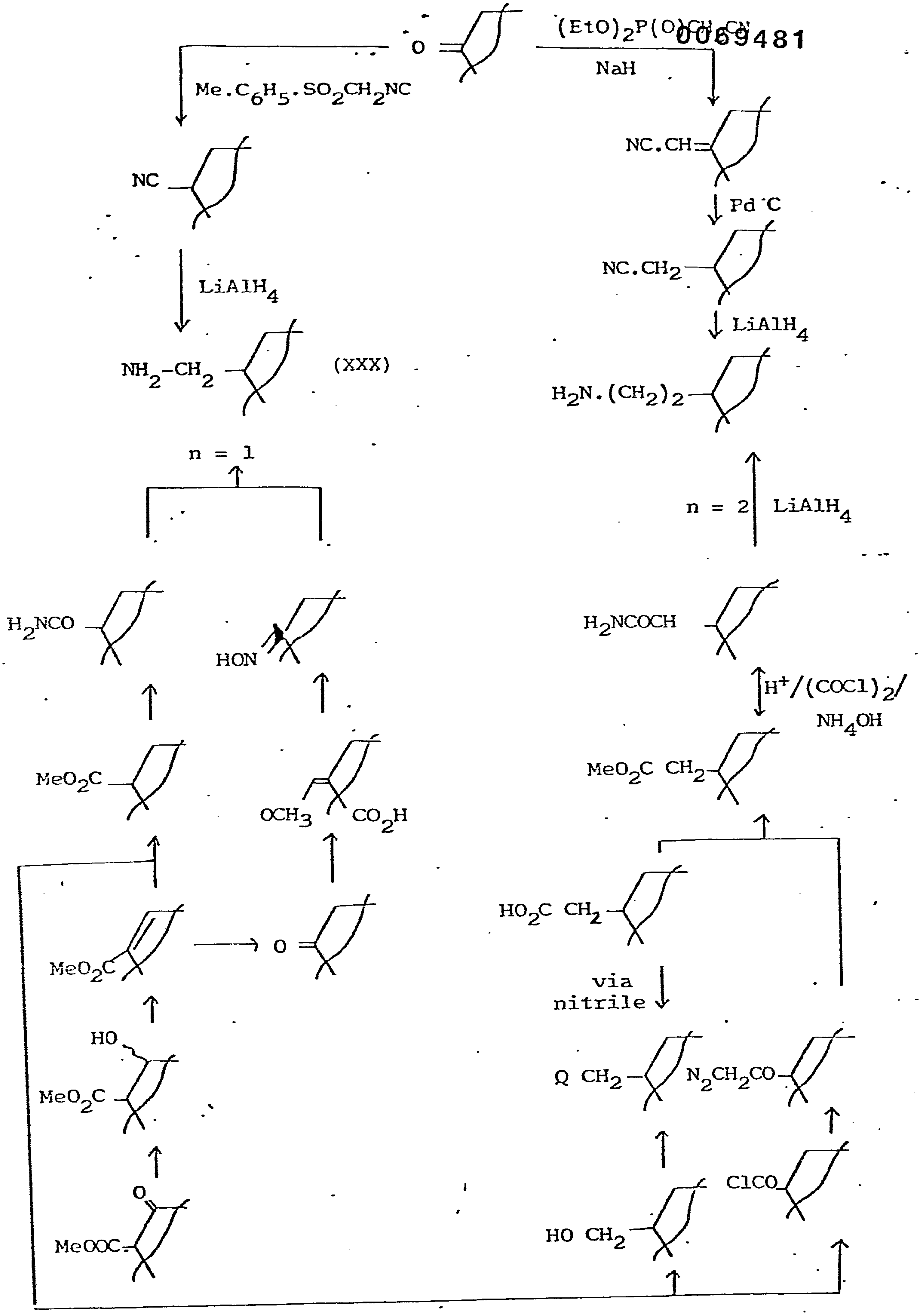
(EtO)$_2$P(O)CH$_2$CN
NaH
Me.C$_6$H$_5$.SO$_2$CH$_2$NC
Pd C
LiAlH$_4$
LiAlH$_4$
(XXX)
n = 1
n = 2
LiAlH$_4$
H$^+$/(COCl)$_2$/
NH$_4$OH
via
nitrile

The acid addition salts of compounds of the formula (I) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_9Y$ as defined. This reaction is suitably carried out in an appropriate solvent such as acetone, methanol, ethanol, dimethylformamide and the like, at ambient or raised temperature and pressure. The nitrogen atom of the moiety of formula (IV) may also form an N-oxide to give an internal N-oxide salt of the compound of the formula (I). The N-oxides may be prepared in conventional manner such as by reaction of the chosen compound of the formula (I) with an organic per-acid, such as m-chloroperbenzoic acid. This reaction is suitably carried out at below-ambient temperature in an organic solvent.

It will be apparent that the product of the reaction of the compounds of formulae (XXIX) and (XXX) will be of formula (XXXI)

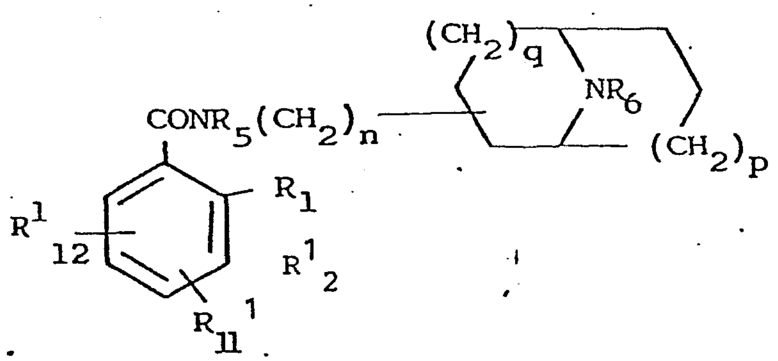

(XXXI)

wherein the variables are as defined in formulae (XXIX) and (XXX). Compounds of the formula (XXXI) containing an $R^1_2$ $R^1_{11}$ or $R^1_{12}$ group convertible to $R_2$, $R_{11}$ or $R_{12}$ are useful intermediates and as such form an important aspect of the invention. Some compounds of the formula (XXXI) will also fall within formula (I).

Compounds of the formula (I) containing an $R_2$, $R_{11}$, $R_{12}$ or $R_6$ group which is convertible to another corresponding group are also useful intermediates and as such form an important aspect of the invention.

By way of example of such conversions, the conversion of a $R_{11}$ nitro group to an $R_{11}$ amino group may be achieved in conventional manner such as by reduction. Thus an optional process step provided by this invention in the preparation of the compounds of the formula (I) substituted by an amino group comprises the reduction of a corresponding intermediate of formula correspondingly substituted by a nitro group.

The reduction of the intermediates containing a nitro group may be effected with reagents known to be suitable for reducing nitroanisole to aminoanisole. A suitable reagent for this reduction is stannous chloride in hydrochloric acid or in mixtures of hydrochloric and acetic acid. The desired amino compound may be obtained from the reaction mixture by neutralisation followed by extraction into a water immiscible solvent such as ethyl acetate from which it may be recovered by evaporation of the solvent.

Another suitable method is catalytic hydrogenation at atmospheric pressure in polar solvent such as ethanol. Transition metal catalysts such as Raney nickel are often used. The desired compound may be obtained from the reaction mixture by filtration and evaporation to dryness.

The initial crude product in both cases may be purified by chromatography or crystallisation or by forming an acid addition salt which may be recrystallised.

In general however, it is more convenient to prepare a compound of the formula (I) containing an amino group from the corresponding $C_{1-7}$ acylamino acid or its reactive derivative, and to deacylate the compound of the formula (I) so formed.

Those compounds of the invention substituted by a $C_{1-7}$ acylamino group may be prepared from the corresponding intermediate containing an amino group by reaction with an acylating derivative, such as previously described as a suitable acylating derivative.
The reaction may proceed as described for the reaction of the compounds of the formula. For a formamido group acylation may be effected with the free acid.

This invention thus also provides an optional process for the preparation of a compound of the formula (I) substituted by an amino group which process comprises the deacylation of a corresponding compound of formula (I) correspondingly substituted by a $C_{1-7}$ acylamino group.

Generally the hydrolysis reaction may be effected by treatment with a base such as an alkali metal hydroxide.

Although as noted $R_{11}$ may be nitro, it is generally preferred that $R_{11}$ is a $R_{11}$ group other than nitro.

$R_6$ optionally substituted benzyl groups may be removed by conventional transition metal catalysed hydrogenolysis to give compounds of the formula (XXXII):

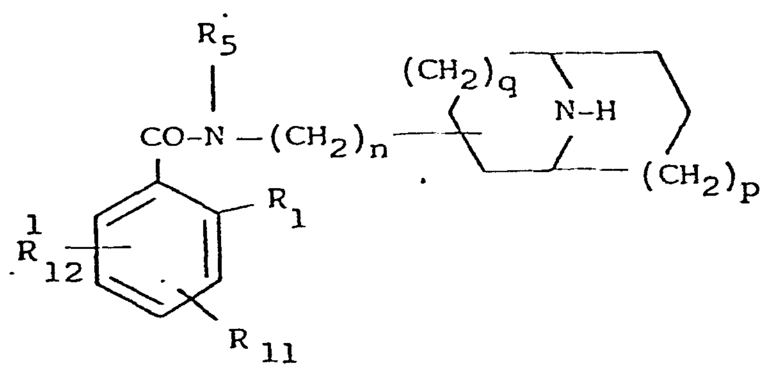

(XXXII)

wherein the variable groups are as defined in formulae and the compound of formula (XXXII) converted into one of formula (I) with a different $R_6$.

This invention also provides an optional process step in the preparation of a compound of the formula (I) which comprises the reaction of a corresponding compound of the formula (XXXII) as hereinbefore defined with a compound $QR_6$ wherein $R_6$ is as defined in formula (I) and Q is a group or atom readily displaced by a nucleophile, converting $R^1_2$, $R^1_{11}$ and $R^1_{12}$ to $R_2$, $R_{11}$ and $R_{12}$ respectively and optionally forming a pharmaceutically acceptable salt or N-oxide of the resulting compound of the formula (I).

Suitable values for Q include Cl, Br, I, $OSO_2CH_3$ or $OSO_2C_6H_4pCH_3$.

Favoured values for Q include Cl, Br and I.

Particularly suitably the compound $QR_6$ is a benzyl halide such as benzyl bromide or benzyl chloride.

The reaction may be carried out under conventional alkylation conditions for example in an inert solvent such as dimethylformamide in the presence of an acid acceptor such as potassium carbonate. Generally the reaction is carried out at a non-extreme temperature such as at ambient or at a slightly elevated temperature.

However, it is generally more convenient to interconvert $R_6$ in the compound of the formula (XXX) before coupling with the compound of the formula (XXIX) or its derivative. Such interconversions are effected conveniently under the above conditions. It is desirable to protect the amine function with a group readily removable by acidolysis such as a $C_{2-7}$ alkanoyl group before $R_6$ interconversion.

$R^1_{12}$ $C_{1-6}$ alkylthio may be converted to $R_{12}$ as defined.

An alternative process according to the present invention thus comprises oxidising a compound of the formula (XXXII) as hereinbefore defined wherein $R_1$ is $C_{1-6}$ alkoxy and one of $R^1_2$, $R^1_{11}$, or $R^1_{12}$ is $C_{1-6}$ alkylthio and thereafter if necessary converting a group $R_2^1$, $R^1_{11}$, $R^1_{12}$ or $R_6$ in the thus formed compound to a group $R_2$, $R_{11}$, $R_{12}$ or $R_6$ as hereinbefore defined; and optionally forming a pharmaceutically acceptable salt of the resultant compound of the formula (I), or N-oxide therof.

These oxidations may conveniently be carried out conventioanlly at below ambient temperatures using a strichiometric amount of an organic peracid in a non-aqueous inert reaction medium preferably a chlorinated hydrocarbon solvent, for example using peracetic acid, or using an inorganic oxidant, such as chromium trioxide in acetic acid.

It will be appreciated by the skilled man that, depending on the other specific substituents in the compound of the formula (I), such an oxidation on a compound of the formula (I) may also form the N-oxide of the bicyclic moiety therein.

Given the specific substitution desired and having been decided whether the compound or its N-oxide is required, the skilled man will readily ascertain whether such interconversion is desirable.

In general however it is more convenient to prepare a compound of formula (I) from the corresponding $C_{1-6}$ alkylsulphinyl acid or its reactive derivative.

Any conversion of $R_2^1$, $R_{11}^1$, $R_{12}^1$ or $R_6$ may take place in any desired or necessary order.

The compounds of the formula (I) are dopamine antagonists.

Depending on their balance between peripheral and central action, the compounds of the formula (I) may be used in the treatment of disorders related to impaired gastro-intestinal motility, such as retarded gastric emptying, dyspepsia, flatulence, oesophagal reflux, peptic ulcer and emesis, and/or in the treatment of disorders of the central nervous system, such as psychosis.

All the compounds of the formula (I) may be used in the treatment of emesis.

The quaternary salts of the compounds of formula (I) are of interest for their beneficial effect on gastric motility.

The compounds of the present invention are dopamine antagonists and depending on their balance between peripheral and central action, may be used in the treatment of disorders relating to impaired gastro-intestinal motility, such as retarded gastric emptying, dyspepsia, flatulence, oesophagal reflux and peptic ulcer,and emesis, and/or in the treatment of disorders of the central nervous system, such as psychosis.

Compounds of the formula (I) wherein $R_1$ is $C_{1-6}$ alkoxy but one of $R_2$, $R_{11}$ and $R_{12}$ is nitro are disclosed in UK Application No. 8019936, and applications claiming priority therefrom, purely as intermediates to the corresponding pharmacologically active amino compounds. It is now believed these compounds of the formula (I) are pharmacologically active per se.

Accordingly the present invention also provides a pharmaceutical composition which comprises a compound of the formula (I) wherein $R_1$ is $C_{1-6}$ alkoxy or $C_{1-6}$ alkylthio, but the third of $R_1$, $R_{11}$ and $R_{12}$ as defined in formula (I) is nitro.

Those compounds of the present invention which are of particular interest for their beneficial effect on gastric motility are the quaternary ammonium salts and N-oxides of compounds of the formula (I).

The invention also provides a pharmaceutical composition comprising a compound of the present invention, or a pharmaceutically acceptable salt thereof, or N-oxide thereof or a solvent adduct of any of the foregoing and a pharmaceutically acceptable carrier.

Such compositions are prepared by admixture and are suitably adapted for oral or parental administration, and as such may be in the form of tablets, capsules, oral liquid preparations, powders, granules, lozenges, reconstitutable powders, injectable and infusable solutions or suspensions or suppositories.  Orally administrable compositions are preferred.

Tablets and capsules for oral administration are usually presented in a unit dose, and contain conventional excipients such as binding agents, fillers, tabletting agents, lubricants, disintegrants and wetting agents. The tablets may be coated according to well known methods in the art. Oral liquid preparations are usually in the form of aqueous or oily suspensions, solutions, emulsions, syrups or elixirs, or are presented as a dry product for reconstitution with water or other suitable vehicle before use. Such liquid preparations may contain conventional additives such as suspending agents, emulsifying agents, non-aqueous vehicles (which may include edible oils), preservatives and flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in the vehicle and filter sterilising before filling into a suitable vial or ampoule and sealing. Advantageously, adjuvants such as local anaesthetic, preservatives and buffering agents are also dissolved in the vehicle.

Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to facilitate uniform distribution of the compound of the invention.

The invention further provides a method of treatment of disorders in mammals, such as humans, which comprises the administration of an effective amount of a compound of the present invention, or a pharmaceutically acceptable salt thereof, or N-oxide thereof or a solvent adduct of any of the foregoing, as hereinbefore defined.

An amount effective to treat the disorders hereinbefore described depends on the relative efficacies of the compounds of the invention, the nature and severity of the disorder being treated and the weight of the mammal. However, a unit dose will normally contain 0.1 to 20mg for example 0.5 to 10mg, of the compound of the invention. Unit doses will normally be administered more than once a day, for example 2, 3, 4, 5 or 6 times a day such that the total daily dose is normally in the range 0.01 to 10mg/kg per day.

The compounds of the present invention have the ability to potentiate the effect of conventional analgesics in migraine treatment when administered concurrently with the analgesic.

Accordingly, the present invention also provides a pharmaceutical composition comprising a compound of the present invention and an analgesic.

The compound of the present invention and the analgesic, such as aspirin or paracetamol, are present in the composition in amounts generally similar to their usual effective dose.

The composition can be a combination product, for example a tablet or capsule containing both a compound of the invention and an analgesic for oral administration, or a twin pack comprising the two active ingredients made up for separate administration.

The invention accordingly provides a method of treatment of migraine comprising the administration of an effective amount of a compound of the invention and an analgesic.

The following examples illustrate the preparation of compounds of the invention and the following descriptions illustrate the preparation of intermediates.

DESCRIPTION 1

2,3-Ethylenedioxy-5-methylsulphinylbenzoic acid (D1)

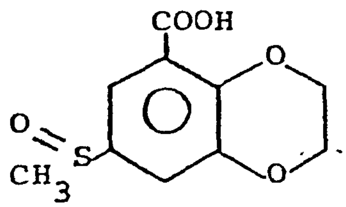

(D1)

A mixture of 2,3-ethylenedioxy-5-methylthiobenzoic acid (6g) and sodium metaperiodate (5.16g) in water (150mls) and methanol (150mls) was stirred at room temperature for 16 hours. The reaction mixture was then poured into ice water and the resulting precipitate was collected via filtration to give 2,3-ethylenedioxy-5-methylsulphinylbenzoic acid (3.6g, 56.3%)

Example 1

2,3-Ethylenedioxy-5-methylsulphinyl-N[3β(8-benzyl-8-azabicyclo{3,2,1}octyl)]benzamide (1)

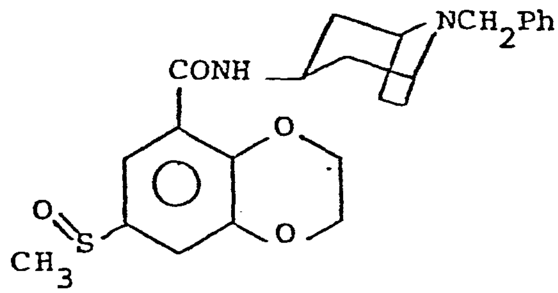

(1)

2,3-Ethylenedioxy-5-methylsulphinylbenzoic acid (0.84g) was dissolved in dry dimethylformamide (25ml), and triethylamine (0.5ml) was added before cooling to 0°C. Ethylchloroformate (0.34ml) was added dropwise to the reaction mixture whilst keeping the temperature at 0°C and the mixture was stirred at this temperature for 15 minutes.

3β-Amino-8-benzyl-8-azabicyclo{3,2,1}octane (0.76g) in dry dimethylformamide (5ml) was added in one portion to the reaction mixture at 0°C before stirring at room temperature for 15 hours.

The solvent was removed in vacuo to leave an oil. Purification via column chromatography gave the title compound as a foam (1.3g, 84%).

Observed mass 440.1801. Required for $C_{24}H_{28}N_2SO_4$ 440.1767.

nmr ($CDCl_3$) τ 2.15 (1H, d, aromatic H), 2.45-2.9 (7H, d and m, aromatic H, -CONH- and $-NCH_2C_6H_5$), 5.4-6.0 (5H, m, $-O-CH_2CH_2-O-$ and 3αH), 6.45 (2H, s, $-NCH_2C_6H_5$), 6.75 (2H, m, bridgehead H's), 7.3 (3H, s, $-SOCH_3$), 7.7-8.6 (8H, m, methylene H's).

Example 2

2,3-Ethylenedioxy-5-methylsulphinyl-N-[3,β-(8-benzyl-8-azabicyclo[3.2,1]octyl)]benzamide (1)

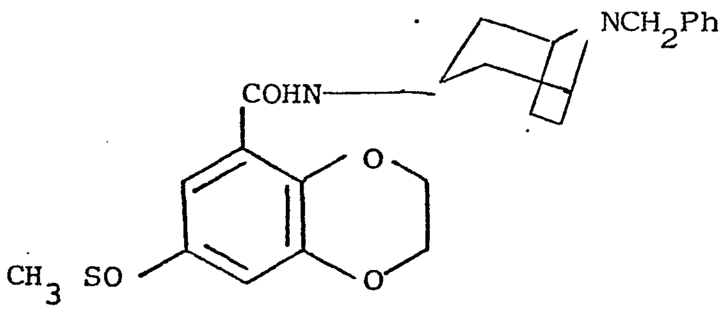

(1)

2,3-Ethylenedioxy-5-methylsulphinylbenzoic acid (1 g) is suspended in dry dichloromethane (30 mls) with oxalyl chloride (0.35 ml) and dry dimethylformamide (0.5 ml) added. The mixture is stirred at room temperature until homogeneous.

The solution is cooled to 0°C and kept below this temperature during the dropwise addition of triethylamine (1.5 mls) in dry dichloromethane (15 mls), followed by the dropwise addition of 3β-amino-8-benzyl-8-azabicyclo-[3.2.1]octane (0.86 g) in dry dichloromethane (15 mls).

The reaction mixture is allowed to warm to room temperature before being shaken with 10% sodium hydroxide solution (10 mls). The organic layer is dried over anhydrous sodium sulphate, filtered and the solvent removed in vacuo to give a colourless oil (2 g) which is purified by column chromatography (silica, 3% methanol in ethyl acetate as eluant) and from ethanol-ether to give (1).

4-chloro-5-methylsulphinyl-2-methoxy-N-[3,β-(8-benzyl-8-azabicyclo-[3.2.1]octyl)]benzamide (2) is prepared analogously

BAD ORIGINAL

## Pharmacological Data

### Increase in intragastric pressure

Intragastric pressure changes were recorded from previously starved conscious but restrained rats using a saline filled catheter inserted into the lumen of the stomach via a permanent gastric fistula. The catheter was connected to a physiological pressure transducer and pressure changes recorded on a hot wire pen recorder. In each animal a pre-dose period of 40 minutes was allowed to obtain a measure of spontaneous activity. An index of activity was obtained by measuring the average height of pressure waves during 10 minute periods. Values for 4 such periods were obtained during assessment of spontaneous activity and for 40 minute period after administration of compound. Student's "t" test was applied to the difference in average values obtained for spontaneous and post compound activity.

Compound (1) significantly increased the index of activity post administration at a dose level of 1mg/kg s.c.

Anti-emetic Activity in the Dog

The compounds were administered subcutaneously 30 minutes prior to administration of a standard dose of apomorphine HCl (0.1 mg/kg subcutaneously) and the vomiting response compared to that obtained when the same animals were dosed with apomorphine HCl and vehicle only. The dose that totally inhibited the vomiting response in 50% or more the dogs was determined. This $ED_{50}$ for Compound 1 was 0.5 mg/kg s.c.

Toxicity

No toxic effects were observed in the above tests.

CLAIMS

1. A compound of the formula (I), or a pharmaceutically acceptable salt or N-oxides thereof, or a solvent adduct of any of the foregoing:

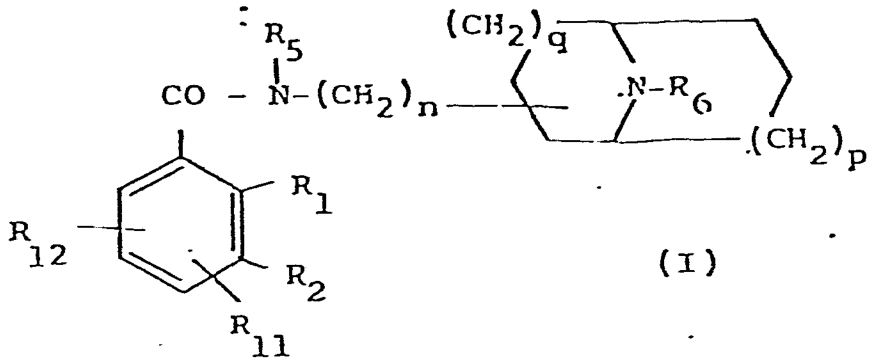

(I)

wherein

n, p and q are independently 0 to 2.

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and

either

$R_1$ is $C_{1-6}$ alkoxy or $C_{1-6}$ alkylthio and one of $R_2$,$R_{11}$ and $R_{12}$ is $C_{1-6}$ alkylsulphinyl and the other two together are $C_{1-2}$ alkylenedioxy, or one of the other two is hydrogen and the other is selected from halogen trifluoromethyl, $C_{1-6}$alkyl,$C_{1-6}$alkoxy,$C_{1-7}$acyl,hydroxy or amino substituted by or aminocarbonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phen $C_{1-4}$ alkyl groups or by $C_{4-5}$ polymethylene;

or

$R_1$ and $R_2$ together are $C_{1-2}$ alkylenedioxy, $R_{12}$ is $C_{1-6}$ alkylsulphinyl and $R_{11}$ is selected from the class of hydrogen, halogen trifluoromethyl, $C_{1-6}$ alkyl, $C_{1-6}$ alkoxy, $C_{1-7}$ acyl, $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phen $C_{1-4}$ alkyl groups or optionally N-disubstituted by $C_{4-5}$ polymethylene.

2. A compound according to claim 1, characterised in that it is of formula (IA):

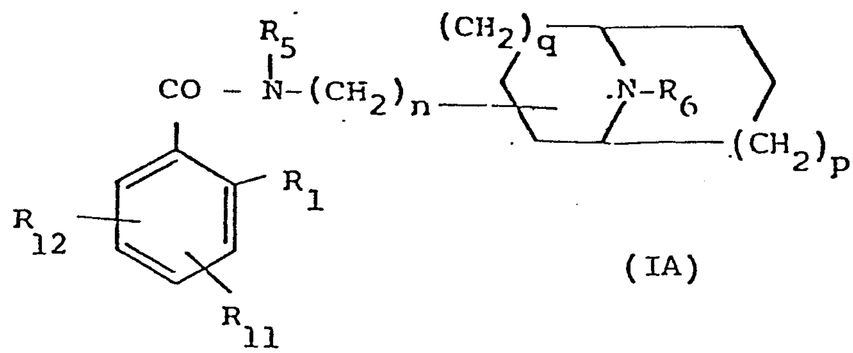

(IA)

wherein:

$R_1$ is $C_{1-6}$ alkoxy or $C_{1-6}$ alkylthio;

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group; and

$R_{11}$ is halogen, $CF_3$, $C_{1-7}$ acyl, aminocarbonyl optionally substituted by one or two $C_{1-6}$ alkyl groups, $C_{1-6}$ alkoxy, $C_{1-6}$ alkylthio, hydroxy or nitro;

$R_{12}$ is $C_{1-6}$ alkylsulphinyl; and

n, p and q are independently 0 to 2.

3. A compound according to claim 1, characterised in that it is of formula (IB):

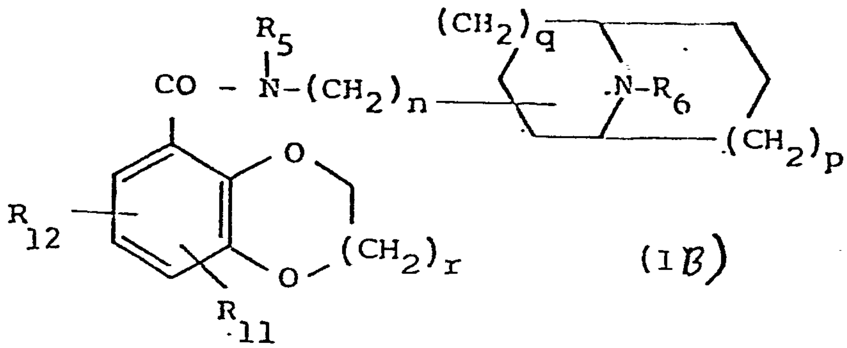

(IB)

wherein:

$R_5$ is hydrogen or $C_{1-6}$ alkyl;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-6}$ alkyl, $C_{1-4}$ alkoxy, trifluoromethyl and halogen, or a thienyl group;

$R_{11}$ is hydrogen, halogen, $CF_3$, $C_{1-7}$ acyl, $C_{1-7}$ acylamino or amino, aminocarbonyl optionally substituted by one or two $C_{1-6}$ alkyl groups, $C_{1-6}$ alkoxy, $C_{1-6}$ alkylthio, hydroxy or nitro.

$R_{12}$ is $C_{1-6}$ alkylsulphinyl; and
n, p, q and r are independently 0 to 2.

4. A compound according to claim 1, characterised in that it is of the formula (XXIII):

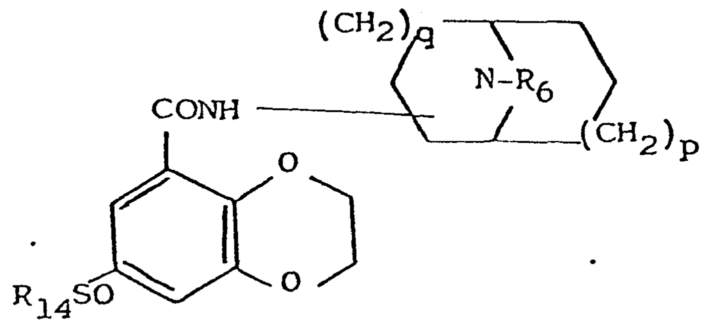

(XXIII)

wherein $R_{14}$ is $C_{1-6}$ alkyl.

5. A compound according to claim 4, characterised in that it is of formula (XXV):

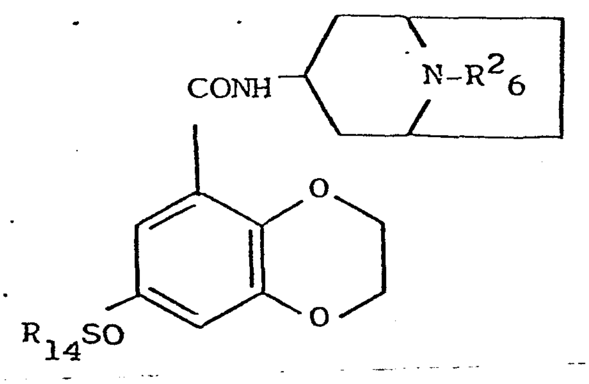

(XXV)

wherein $R^2{}_6$ is $C_{5-7}$ alkyl; a group $-(CH_2)_tR^1{}_8$ wherein t is 1 or 2 and $R^1{}_8$ is optionally substituted phenyl as defined in claim 1; cylohexylmethyl, or thienylmethyl.

6. 2,3-Ethylenedioxy-5-methylsulphinyl-N[3β(8-benzyl-8-azabicyclo[3.2.1]octyl)benzamide

7. A process for the preparation of a compound according to claim 1, which process comprises reacting an acid of the formula (XXIX):

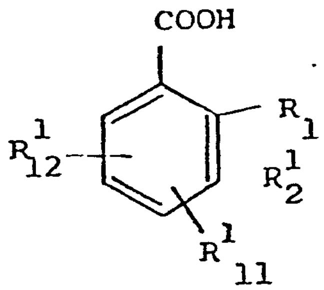

(XXIX)

wherein $R_1$ is as defined; and $R^1_2$, $R^1_{11}$ and $R^1_{12}$ are $R_2$, $R_{11}$ and $R_{12}$ as defined but when $R_1$ is $C_{1-6}$ alkoxy the third of them may be $C_{1-6}$ alkylthio; or a reactive derivative thereof, with a compound of formula (XXX).

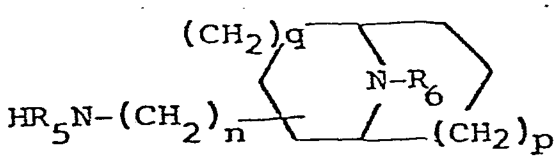

(XXX)

wherein $R_6$ is as defined in claim 1, the remaining variable groups being as defined in claim 1, and thereafter if necessary converting a group $R^1_2$, $R^1_{11}$ or $R^1_{12}$ in the thus formed compound to another group $R_2$, $R_{11}$ or $R_{12}$ respectively; converting $R_6$ to another $R_6$; and optionally forming a pharmaceutically acceptable salt of the resultant compound of for the formula (I).

8. A pharmaceutical composition comprising a compound according to claim 1, or a pharmaceutically acceptable salt thereof, or N-oxide thereof or a solvent adduct of any of the foregoing and a pharmaceutically acceptable carrier.

9. A compound according to claim 1, or a pharmaceutically acceptable salt or N-oxide thereof or a solvent adduct of any of the foregoing for use in the treatment of emesis and/or disorders related to impaired gastro-intestinal motility.

10. A method of treatment of emesis and/or disorders related to impaired gastro-intestinal motility in mammals, such as humans, which comprises the administration of an effective amount of a compound according to claim 1, or a pharmaceutically acceptable salt thereof, or N-oxide thereof or a solvent adduct of any of the foregoing.

European Patent Office

# EUROPEAN SEARCH REPORT

0069481

Application number

EP 82 30 3055

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | FR-A-2 446 823 (DELALANDE) *The whole document* --- | 1-2,7-10 | C 07 D 451/04<br>C 07 D 451/02<br>C 07 D 451/14<br>A 61 K 31/46 //<br>C 07 D 319/18 |
| A | GB-A-2 042 522 (DELALANDE) *The whole document* --- | 1-2,7-10 | |
| D,A | EP-A-0 013 138 (BEECHAM) *The whole document* --- | 1-2,7-10 | |
| P,X | EP-A-0 042 705 (BEECHAM) *The whole document* --- | 1-2,7-10 | |
| P,A | EP-A-0 031 219 (BEECHAM) *The whole document* --- | 1-10 | TECHNICAL FIELDS SEARCHED (Int. Cl. 3) |
| P,A | FR-A-2 476 088 (DELALANDE) *The whole document* ----- | 1-2,7-10 | C 07 D 451/00<br>A 61 K 31/00 |

The present search report has been drawn up for all claims

| Place of search | Date of completion of the search | Examiner |
|---|---|---|
| THE HAGUE | 23-09-1982 | NUYTS A.M.K.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82